(19) Europäisches Patentamt
European Patent Office
Office européen des brevets

(11) Publication number: **0 601 187 A1**

(12) **EUROPEAN PATENT APPLICATION
published in accordance with Art.
158(3) EPC**

(21) Application number: **93904361.8**

(22) Date of filing: **26.02.93**

(86) International application number:
**PCT/JP93/00246**

(87) International publication number:
**WO 93/20109 (14.10.93 93/25)**

(51) Int. Cl.[5]: **C07K 15/04**, A61K 37/02, C12P 21/02, //(C12P21/02, C12R1:10)

A request for correction (sequence listing) has been filed pursuant toR. 88 EPC. A decision will be takendduring the proceedings before the Examining Division (Guidelines for Examination, A. V,2.2).

(30) Priority: **31.03.92 JP 103890/92**
**31.03.92 JP 103946/92**
**31.03.92 JP 103954/92**

(43) Date of publication of application:
**15.06.94 Bulletin 94/24**

(84) Designated Contracting States:
**BE FR**

(71) Applicant: **HIGETA SHOYU CO., LTD.**
**2-3, Nihonbashikoamicho**
**Chuo-ku, Tokyo 103(JP)**

(72) Inventor: **TANAKA, Yasushi**
**6-14, Sangencho**
**Choshi-shi, Chiba-ken 288(JP)**
Inventor: **UCHIDA, Yasushi**
**9-25, Nakanotatemachi**
**Saga-shi, Saga-ken 840(JP)**
Inventor: **HASEGAWA, Kaname**
**394-9, Kasugacho**
**Choshi-shi, Chiba-ken 288(JP)**
Inventor: **KADOWAKI, Kiyoshi**
**3094-3, Kasugacho**
**Choshi-shi, Chiba-ken 288(JP)**

(74) Representative: **Clisci, Serge et al**
**S.A. FEDIT-LORIOT & AUTRES**
**CONSEILS EN PROPRIETE INDUSTRIELLE**
**38, Avenue Hoche**
**F-75008 Paris (FR)**

# (54) NOVEL ANTIBIOTIC AND PRODUCTION AND USE THEREOF.

(57) A novel antifungal antibiotic represented by the following formula: X-Asn-Ser-Pro-Asn-Tyr-Asn-Gln wherein X represents an α-amino fatty acid residue. HUT57A and HUT57B, two components of this antibiotic, have been isolated from a culture of *Bacillus licheniformis* UTK. Both are novel substances and useful as antifungal agents not only for man and animal but also for agrohorticultural use.

EP 0 601 187 A1

## DETAILED EXPLANATION OF THE INVENTION

The present invention relates to novel antibiotics effective as a remedy for infectious diseases to be caused by fungi and also to a method of producing them and to use of them.

## PRIOR ART

Heretofore, infectious diseases to be caused by fungi such as mold, yeast and others tend to increase not only in local infection in the skin, respiratory organs, vagina and others but also in systemic infection.

In particular, in these days, not only superficial mycosis such as typically athlete's foot and the like but also systemic infectious diseases such as profound infectious diseases to be caused by depression of immunological competence resulting from use of immunosuppressive agents, carcinostatics and others are increasing. Many cases of fatal serious infectious diseases have been reported in the clinical medicine (Nippon Byori Gakkai Kaishi 74, 61 (1985)).

However, as compared with noticeable progress of the chemotherapy for infectious diseases to be caused by bacteria, it must be said that the chemotherapy for infectious diseases to be caused by fungi is belated at present. One reason is that bacteria are prokaryocytes, being different from animal cells of eukaryocytes with respect to the cell background, and preparation of medicines having selective toxicity to them is easy; while fungi which are composed of eukaryocytes like animal cells are hardly differentiated from animal cells because of the similar cell background of them so that elevation of the selective toxicity specific only to fungi but not to animal cells is not easy.

At present, only amphotericin B is known as a medicine effectively applicable to profound mycosis (candidiasis, aspergillosis, cryptococcosis, mucormycosis) (Annu. Rev. Pharmacol., Toxicol., 23, 303 (1983)-). Since fungi are parasitic in the inside of the corneum of the skin in superficial mycosis such as athlete's foot to be chronic, the disease once cured by the current medicines often recurs in many cases. Development of novel antifungal which are effective to such various mycosis and which have a high selective toxicity only to the fungi to cause the diseases is desired.

## PROBLEMS TO BE SOLVED BY THE INVENTION

The present invention has been made so as to satisfy the above-mentioned request in this technical field, and the object of the present invention is to develop excellent novel substances which are useful as preventives and remedies to mycosis, which have a high activity and a high selective toxicity only to fungi and which are highly safety.

## MEANS FOR SOLVING THE PROBLEMS

The present inventors made screening of known chemical substances so as to attain the above-mentioned object but in vain.

Under the situation, the present inventors paying their attention to natural substances, especially to fermentation products by microorganisms in view of the safety to human bodies, investigated various microorganisms and, as a result, have found that Bacillus sp. UTK (FERM P-11442, FERM BP-3794; this has been identified to be Bacillus licheniformis UTK at present), which had been successfully isolated by the present inventors, may produce novel compounds to be represented by the following formula in the culture.

X-Asn-Ser-Pro-Asn-Tyr-Asn-Gln

where X represents an $\alpha$-amino-fatty acid (see SEQ ID No: 1).

They further studied in detail the novel compounds and have identified novel antibiotics HUT57A and HUT57B, establishing the method of producing them. They also have identified the excellent antifungal effect of the substances. Accordingly, they have completed the present invention.

## BRIEF EXPLANATION OF THE DRAWINGS

Fig. 1 shows an ultraviolet absorption spectrum of HUT57A.

Fig. 2 shows an infrared absorption spectrum of HUT57A.

Fig. 3 shows an ultraviolet absorption spectrum of HUT57B.

Fig. 4 shows an infrared absorption spectrum of HUT57B.

HUT57A of the present invention has the following physico-chemical properties.

Physico-chemical Properties of HUT57A

1)

| | |
|---|---|
| Elementary Analysis: | C: 55.1<br>H: 7.2<br>N: 16.1 |

2) Melting Point: 128 ° C
3) Specific Rotatory Power: $[\alpha]^{20}_D$ -79.3 (methanol)
4) Ultraviolet Absorption Spectrum: Fig. 1 (absorption is admitted in the range of from 220 to 400 nm.)
5) Infrared Absorption Spectrum: Fig. 2
6) Solubility in Solvents:

| | |
|---|---|
| Methanol | + |
| Ethanol | + |
| N-butanol | - |
| Acetone | - |
| Alkaline Water (pH 7.5 to 9.0) | + |
| Water | + |
| Ethyl Acetate | - |
| Chloroform | - |

7) Color Reaction

| | |
|---|---|
| Biuret Reaction | + |
| Ninhydrin Reaction | +/- |
| Millon Reaction | + |
| Indole-Hydrochloric Acid Reaction | + |
| Molisch Reaction | - |
| Tollens Reaction | - |
| Sakaguchi Reaction | - |
| Orcinol-Sulfuric Acid Reaction | - |
| Cysteine-Sulfuric Acid Reaction | - |

8) Differentiation in acidic, neutral and basic properties: Neutral
9) Color of Substance: White
10) Hemolyzation Activity: -
11) Molecular Weight: (FAB-MS) m/z
1043 (M + H), 1065 (M + Na)
12) This is a compound to be represented by the following formula:
X-Asn-Ser-Pro-Asn-Tyr-Asn-Gln
where X represents an $\alpha$-amino-fatty acid of the following formula:

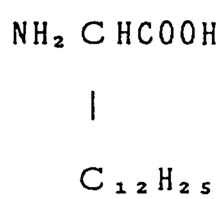

(see SEQ ID No: 2).

HUT57B of the present invention has the following physico-chemical properties.

Physico-chemical Properties of HUT57B

1)

| | |
|---|---|
| Elementary Analysis: | C: 55.6<br>H: 7.3<br>N: 15.7 |

2) Melting Point: 128 °C
3) Specific Rotatory Power: $[\alpha]^{20}_{D}$ -78.5 (methanol)
4) Ultraviolet Absorption Spectrum: Fig. 3 (absorption is admitted in the range of from 220 to 400 nm.)
5) Infrared Absorption Spectrum: Fig. 4
6) Solubility in Solvents:

| | |
|---|---|
| Methanol | + |
| Ethanol | + |
| N-butanol | - |
| Acetone | - |
| Alkaline Water (pH 7.5 to 9.0) | + |
| Water | + |
| Ethyl Acetate | - |
| Chloroform | - |

7) Color Reaction

| | |
|---|---|
| Biuret Reaction | + |
| Ninhydrin Reaction | +/- |
| Millon Reaction | + |
| Indole-Hydrochloric Acid Reaction | + |
| Molisch Reaction | - |
| Tollens Reaction | - |
| Sakaguchi Reaction | - |
| Orcinol-Sulfuric Acid Reaction | - |
| Cysteine-Sulfuric Acid Reaction | - |

8) Differentiation in acidic, neutral and basic properties: Neutral
9) Color of Substance: White
10) Hemolyzation Activity: -
11) Molecular Weight: (FAB-MS) m/z
1057 (M+H), 1079 (M+Na)
12) This is a compound to be represented by the following formula:
X-Asn-Ser-Pro-Asn-Tyr-Asn-Gln
where X represents an $\alpha$-amino-fatty acid of the following formula:

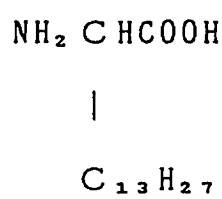

(see SEQ ID No 3).

It is to be understood that production of HUT57A or/and B is not restricted to only the use of the particular microorganisms as described herein. The present invention is directed to use of not only the

described microorganisms but also all other mutants of them capable of producing HUT57A or/and B, including artificial mutants of the described microorganisms to be obtained by mutation by X-ray irradiation or ultraviolet irradiation thereto or by treatment thereof with N-methyl-N'-nitro-N-nitrosoguanidine, 2-aminopurine or the like and also natural mutants of them.

Next, the microbiological properties of Bacillus licheniformis UTK are mentioned below.

Microbiological Properties of Bacillus licheniformis UTK

(A) Morphological Properties (in broth-agar medium):

| | |
|---|---|
| Cell Size: | 3μ to 5μ × 0.5μ to 0.9μ |
| Cell Shape: | bacillus form |
| Polymorphism of Cell: | Negative |
| Motility: | Positive |
| Sporulation: | Positive |
| Gram Stain: | Positive after incubation for 18 to 48 hours |

(B) Physiological Properties:

| | |
|---|---|
| Catalase | + |
| Oxidase | + |
| Methyl Red | - |
| V-P Test (pH 5.5) | + |
| Liquefaction of Gelatin | + |
| Hydrolysis of Starch | + |
| Urease | - |
| Utilization of Citric Acid, Christensen | + |
| Formation of Hydrogen Sulfide, TSI | + |
| Formation of Indole | + |
| Denitrification | + |
| Reduction of Nitrites | - |
| O-F Test: | fermented |
| Influence by Oxygen: | aerobic |
| Heat Resistance: | 90 °C |
| pH for Growth: | pH 5 to 9 |

Formation of Acid and Gas from Saccharides:

| Saccharides | Formation of Gas | Formation of Acid |
|---|---|---|
| Glucose | - | + |
| Mannose | - | + |
| Sucrose | - | + |
| Rhamnose | - | - |
| Lactose | - | +/- |
| Maltose | - | +/- |
| Cellobiose | - | + |
| Raffinose | - | - |
| Starch | - | + |
| Glycerin | - | +/- |
| Sorbitol | - | + |
| Inositol | - | - |
| NaCl tolerance NaCl 5% | + | |
| NaCl 7 % | + | |

(C) Characteristic:

This accumulates chitosanase UTK in the culture.

Novel antibiotics HUT57A or/and B of the present invention may be produced by inoculating microorganisms of producing them, which belong to the genus Bacillus, for example; Bacillus licheniformis UTK in a nutrient medium containing carbon and nitrogen sources to be assimilated by them followed by incubating them therein under an aerobic condition, for example, by shaking culture or aerial stirring culture.

As carbon sources, preferably used are glucose, sucrose, starch, fructose, glycerin and other carbohydrate.

As nitrogen sources, preferably used are oat meal, yeast extract, peptone, gluten meal, cotton seed meal, soybean meal, corn steep liquor, dry yeast, wheat germ, peanut meal, chicken bone meal and others. Also advantageously usable are inorganic and organic nitrogen compounds, for example, ammonium salts (such as ammonium nitrate, ammonium sulfate, ammonium phosphate and others), urea, amino acids and others.

Use of combination of such carbon sources and nitrogen sources is advantageous, but it is not always necessary to use only pure ones. This is because impure ones often contain growth factors or micro nutrients.

If desired, inorganic salts such as those mentioned below may be added to the medium. They are, for example, sodium carbonate, potassium carbonate, sodium phosphate, potassium phosphate, sodium chloride, potassium chloride, sodium iodide, potassium iodide, magnesium salts, copper salts, cobalt salts and others.

In particular, where the medium is a strongly foaming one, liquid paraffin, animal oils, vegetable oils, mineral oils, silicones, higher alcohols and the like may be added thereto, if desired.

For industrial mass-production of the intended product, the microorganisms are preferably incubated by aerial stirring culture like the case of producing other fermentation products. Where a small amount of the intended product is produced, they are preferably incubated by shaking culture in a flask.

Where the incubation is effected in a large-size tank, it is preferred that the antibiotic-producing microorganisms are first inoculated and cultivated in a relatively small amount of a medium and thereafter the resulting culture is transferred to a large-size production tank in which the previously grown microorganisms are further incubated to produce the intended antibiotic(s), for the purpose of preventing their delay in growing and producing HUT57A or/and B. In the case, the composition of the medium to be used in the pre-culture and that of the medium to be used for the latter incubation to produce the antibiotic(s) may be the same one, or if desired, they may be different from each other.

The incubation is preferably effected under the condition of aerial stirring. For example, any known method, such as stirring culture with propellers or other mechanical stirrers, rotary or shaking culture in a fermenter, pumping or air-blowing culture or the like, may suitably be employed. Air to be used for aeration

of the culture system is desired to be previously sterilized.

The incubation temperature may suitably be varied within the range where the antibiotic-producing microorganisms may produce the present substance(s) of HUT57A or/and B. In general, the incubation may be effected at 10 to 40 °C, preferably from 25 to 35 °C. The incubation time varies, depending upon the incubation condition and the amount for fermentation, and it is generally approximately from one day to one week.

After completion of the fermentation, the intended HUT57A or/and B is/are recovered from the culture. Briefly, the cultivated microorganismic cells are directly or after having been extracted with water and/or organic solvent(s), disrupted mechanically or ultrasonically or by any other known means and thereafter extracted with water and/or organic solvent(s). Then, the product is recovered and purified by ordinary methods. The culture may directly be extracted with organic solvent(s) or, alternatively, it may be filtered through a filter membrane or the like. If desired, the culture may be brought into contact with an active charcoal, powdery cellulose, adsorbing resin or the like carrier so that the produced HUT57A or/and a is/are adsorbed to the carrier, and thereafter the product may be desorbed therefrom by elution.

For isolating and purifying HUT57A or B, any ordinary method for harvesting antibiotics may be employed. For instance, usable are solvent extraction with water, organic solvents or mixed solvents of them; chromatography; recrystallization from a single solvent or a mixed solvent; and other ordinary means. The may be employed singly or in combination of them.

Recovery and purification of HUT57A or B may suitably be effected by known methods, as mentioned above. If desired, the following method, as one example, may also be employed. First, the culture is made acidic and then extracted with methanol. Ethyl acetate is added to the resulting extract, the formed precipitates are collected and dissolved in methanol, the solution is subjected to chromatography or recrystallization, and the thus purified product may be freeze-dried, if desired. In this way, the intended antifungal substance HUT57A or B may be isolated.

Alternatively, the following method, as still another example, may also be employed. The culture is filtered through a filter membrane so that the precipitates containing the cultivated cells are collected on the membrane, and they are extracted with methanol so as to collect a methanol fraction containing HUT57A or B. The fraction may then be subjected to chromatography or recrystallization and, if desired, freeze-dried. In this way, the intended HUT57A or B may be isolated. Again, the extraction may be effected with isopropanol in place of methanol, and the extract may then be subjected to chromatography.

Where the compound(s) of the present invention HUT57A or/and B is/are used as a medicine, it may be administered to animals including human either directly or as a drug composition containing it in an amount of, for example, from 0.1 % to 99.5 %, preferably from 0.5 % to 90 %, in a pharmaceutically acceptable non-toxic and inactive carrier.

As the carrier, usable are one or more solid, semi-solid or liquid diluting agents, fillers and other ordinary drug additives. The drug composition is desired to be administered as its dose unit. The drug composition of the present invention may be administered by oral administration, tissue administration, local administration (such as endermic administration, etc.) or rectal administration. It may also be used for external application. Needless to say, the drug composition is administered as a form suitable to the administration route.

It is desired that the amount of the antifungal to be used is adjusted in consideration of the condition of the patient, such as its age or body weight, the administration route, the condition and degree of the disease and others. In general, the amount to be administered to one adult is from 10 to 2000 mg a day, as the active ingredient of the present invention. As the case may be, the necessary amount may be smaller than the range or may be larger than the same. If a large amount of the medicine is administered, it is desired that the total amount thereof a day is divided into several parts for separate administration in one day.

Oral administration of the medicine may be effected, using its solid or liquid dose unit of, for example, powder, powder mixture, tablets, dragées, capsules, drops, sublingual tablets and other preparation forms.

Powder of the medicine may be produced by grinding the active substance into a desired fineness. Powder mixture of it may be produced by grinding the active substance into a desired fineness followed by blending the resulting powder with a fine powdery drug carrier, such as a fine powdery edible carbohydrate of, for example, starch, mannitol or the like. If desired, a flavoring, a preservative, a dispersing agent, a colorant, a perfume and other additives may be added to the preparation.

Capsules of the medicine may be produced by encapsulating powder, powder mixture or granules of the medicine with a capsule coat such as a gelatin capsule coat. Prior to the encapsulation, a lubricant or fluidizing agent, such as colloidal silica, talc, magnesium stearate, calcium stearate, solid polyethylene glycol or the like powdery substance may be incorporated into the powdery or granular medicine. Addition

of a disintegrator or a solubilizer, for example, carboxymethyl cellulose, calcium carboxymethyl cellulose, hydroxypropyl cellulose of low substitution degree, calcium carbonate or sodium carbonate, to the medicine preparation to be encapsulated is recommended so as to elevate the effectiveness of the medicine when the capsules have been ingested.

If desired, the fine powdery medicine of the present invention may be suspended and dispersed in a vegetable oil along with polyethylene glycol, glycerin and a surfactant and the resulting dispersion may be wrapped with a gelatin sheet to give soft capsules.

Tablets of the medicine may be produced by granulating or slagging the active substance containing powder mixture followed by adding a disintegrator or lubricant thereto and forming the resulting blend into tablets.

The powder mixture comprises a suitably powdered active substance and the above-mentioned diluting agent and base and may optionally contain a binder (for example, sodium carboxymethyl cellulose, alginates, gelatin, polyvinyl pyrrolidone, polyvinyl alcohol, etc), a slow-releasing agent (for example, paraffin, etc.), a re-absorbing agent (for example, quaternary salts) and/or an adsorbent (for example, bentonite, kaolin, dicalcium phosphate, etc.). The powder mixture may be wetted with a binder such as syrup, starch paste, gum arabic, cellulose solution or polymer substance solution and then forcedly sieved to give granules. In place of forming granules from the powder in this manner, the powder may directly be processed with a tabletting machine and thereafter the resulting slugs of an incomplete form may be pulverized to granules.

A lubricant such as stearic acid, stearates, talc, mineral oils and others may be added to the granules to be obtained in this manner, so as to prevent their adhesion to each other. The lubricant-containing mixture is then formed into tablets. If desired, the active substance may directly be formed into tablets, after having been combined with a fluid inactive carrier, without taking the above-mentioned granulating or slagging step. As the case may be, a transparent or semi-transparent protective film composed of a shellac sealant film, or a sugar or polymer material film, or a waxy lubricant top coat film may be employed for overcoating the tablets.

Other oral preparations, such as solution, syrup, elixir and the like may also be produced in the unit dose form of containing a determined amount of the active substance. Syrup may be produced by dissolving the active compound in a suitable aromatizing aqueous solution; and elixir may be produced by blending it with a non-toxic alcoholic carrier. Suspension may be formulated by dispersing the active compound in a non-toxic carrier. A solubilizer and an emulsifier (for example, ethoxylated isostearyl alcohols, polyoxyethylene sorbitol esters), preservatives, aromatizing agents (such as peppermint oil, saccharin) and others may optionally be added to them.

If desired, the unit dose formulation for oral administration may be encapsulated into microcapsules. The formulation may be coated with a coating film or may be embedded into polymer substances, wax or the like so as to prolong the active life of the active substance or to attain slow release of the active substance.

Parenteral administration may be effected, using a liquid dose unit form of, for example, a solution or suspension for subcutaneous, intramuscular or intravenous injection. This may be prepared by dissolving or suspending a determined amount of the active compound in a non-toxic liquid carrier suitable for injection of, for example, an aqueous or oily medium followed by sterilizing the resulting solution or suspension. Alternatively, a determined amount of the active compound may be put in a vial and thereafter the vial may be sterilized along with the content and then sealed. For dissolution or mixing of the powdery or freeze-dried active compound just before its administration, a preparatory vial or carrier may be prepared along with it. For making the injection containing the active compound isotonic, a non-toxic salt or salt solution may be added thereto. If desired, a stabilizer, a preservative, an emulsifier and the like may also be added thereto.

Rectal administration may be effected, using a suppository composed of the active compound and a low melting point solid of, for example, polyethylene glycol, cacao butter, higher esters (e.g., myristyl palmitate) or a mixture of them.

The effectiveness of the substances of the present invention as a medicine may be identified by various tests.

The antimicrobial spectra of HUT57A and HUT57B to be in the substances of the present invention may be obtained, for example, from the respective minimum growth inhibiting concentration (MIC) of HUT57A and HUT57B to various fungi such as clinically isolated strains, animal-infected fungi, phytopathogenic fungi and others to be obtained on the basis of the method as designated by Japan Chemotherapy Association.

Identification of the safety of HUT57A and HUT57B may be effected by examining the toxicity, if any, of them. For instance, employable for the purpose are a cytotoxicity test using animal cells and an acute

toxicity test (oral single administration, intravenous single injection, intravenous repeated injection) using mice.

As one example of the test of testing the effectiveness of the active substances, HUT57A or HUT57B may be administered to systemically fungi-infected mice, to which various clinically isolated strains (Candida albicans IFM4009, Aspergillus fumigatus Tsukuba No. 12, Cryptococcus neoformans 145A) were intravenously inoculated, whereby the effectiveness of administered the substance may be examined. The test results are shown in Tables 1 to 3 below.

Table 1 - Number of Living Mice as Infected with C. albicans IFM4009

| | Test Period (day) | | | | | | | | | | | | | | |
|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 |
| Control | 10 | 10 | 4 | 2 | 2 | 2 | 2 | 2 | 1 | 1 | 1 | 0 | 0 | 0 | 0 |
| HUT57A 60 mg/kg | 10 | 10 | 5 | 5 | 5 | 5 | 3 | 3 | 3 | 2 | 1 | 1 | 0 | 0 | 0 |
| HUT57B 60 mg/kg | 10 | 10 | 10 | 10 | 9 | 9 | 8 | 5 | 5 | 5 | 5 | 4 | 3 | 3 | 3 |

Table 2 - Number of Living Mice as Infected with A. fumigatus Tsukuba No. 12

| | Test Period (day) | | | | | | | | | | | | | | |
|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 |
| Control | 10 | 10 | 10 | 10 | 10 | 9 | 8 | 6 | 4 | 3 | 3 | 3 | 3 | 3 | 3 |
| HUT57A 25 mg/kg | 10 | 10 | 10 | 10 | 10 | 10 | 9 | 7 | 5 | 4 | 4 | 3 | 3 | 3 | 3 |
| HUT57B 25 mg/kg | 10 | 10 | 10 | 10 | 10 | 10 | 10 | 10 | 10 | 9 | 9 | 9 | 9 | 9 | 9 |

Table 3 - Number of Living Mice as Infected with C. neoformans 145A

| | Test Period (day) | | | | | | | | | | | | | | |
|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 |
| Control | 10 | 10 | 4 | 4 | 4 | 4 | 4 | 4 | 2 | 0 | 0 | 0 | 0 | 0 | 0 |
| HUT57A 10 mg/kg | 10 | 10 | 8 | 8 | 8 | 7 | 7 | 7 | 5 | 3 | 2 | 1 | 1 | 1 | 1 |
| HUT57B 10 mg/kg | 10 | 10 | 6 | 5 | 5 | 5 | 5 | 5 | 5 | 5 | 3 | 1 | 0 | 0 | 0 |

From the results, it is understood that HUT57A and HUT57B both have a selective toxicity to fungi and strongly affect various pathogenic fungi, that the toxicity of them is extremely weak and that they are effective to systemic mycosis in mice. Therefore, it has strongly been suggested that the substances of the present invention are effective as a remedy for various fungi-infected diseases such as candidiasis, aspergillosis, cryptococcosis, mucormycosis, etc.

HUT57A or/and B is/are utilizable as excellent preventive and/or remedial medicines to fungi, and additionally they may also be utilized as preventive and/or remedial drugs for various agricultural and horticultural diseases to be caused by fungi.

The agricultural and horticultural fungicide of the present invention contains the novel antibiotic(s) HUT57A or/and B as an active ingredient and may be formulated along with various carriers by any ordinary method of formulating agricultural drugs. For instance, it may be formulated as a liquid composition, a wettable powder, a powder, an emulsion or the like. The agricultural drug compositions or some external medicines may contain either pure antibiotic(s) HUT57A or/and B of the present invention or crude products containing them. For instance, they may contain the culture of the microorganisms of producing the active substance or its concentrate, directly as an active ingredient.

The agricultural and horticultural fungicide of the present invention may be used broadly for exterminating phytopathogenic fungi. As demonstrated in the following examples, it displayed an excellent antifungal property. In addition, even though it was sprayed over crop plants by an ordinary method, it caused no chemical injury to them. Further, it is free from chemical injury to not only human and domestic animals but also fishes, and therefore it may freely and broadly be used as a safe agricultural chemical composition not only in crop fields but also in forests and for domestic use.

The agricultural and horticultural fungicide of the present invention is effective broadly for preventing and curing plant diseases to be caused by fungi. For instance, it is unlimitedly effective to various plant diseases to be caused by, for example, blast (Pyriculalia oryzae); various gray mold and others (Botrytis cinerea); yellows, Fusarium wilt, dry rot, dead arm, for various agricultural and horticultured crops, damping - off for trees (Fusarium oxysporum); scab for wheat and others (Gibberella zeae), etc.

Next, the present invention will be explained in more detail by way of the following examples.

Example 1

(1) Fermentation and Production:

Cells of Bacillus licheniformis UTK (FERM BP-3794) were inoculated in a basic medium (pH 7.2) comprising 2 % of fructose, 1 % of Polypeptone S (by Nippon Pharmaceutical Co.), 0.1 % of yeast extract (by Oriental Yeast Industry Co.), 0.01 % of CaCO3, 0.01 % of NaCl and 300 ml of Einol (by Biott Co.) and incubated in a 1000-liter tank at 30 °C. After 12 hours, one liter of Einol was added thereto and incubation was continued for further 24 hours with introducing air of 280 liter/min thereinto at agitation of 40 rpm.

(2)-1 Recovery and Purification:

Hydrochloric acid was added to the thus obtained culture broth so that the pH of the broth was adjusted to be 2.0 to 3.0. The precipitates formed were extracted with methanol, the methanol extract was concentrated in vacuum, the supernatant was extracted with acetone, the acetone extract was concentrated in vacuum, ethyl acetate was added to the supernatant, and the precipitates formed were dissolved in methanol.

The methanol solution was subjected to column chromatography with Toyopearl HW40f, eluting with methanol, and the active fraction thus eluted was concentrated in vacuum. This was then subjected to column chromatography with Sephadex LH-20, eluting with methanol, and the active fraction was processed by high performance liquid chromatography (HPLC) (column: Capcell Pack C18 of 15 mm diameter × 250 mm length; mobile phase: 100 mM triethylamine-acetic acid buffer (pH 7.2)/methanol of 50/50 to 100/0; detection: UV 276 nm). The active fraction was again subjected to chromatography to separate into two components, and antibiotics HUT57A and HUT57B were obtained.

(2)-2 Recovery and Purification:

The obtained culture was concentrated by filtering through a filter membrane (produced by Millipore Co.; 0.45 μm prostak) and washed fully with water, and 80 % methanol was added thereto to extract the active substance. The resulting extract was diluted with water so that the methanol concentration was lowered to 40 %, and the precipitates formed were removed by filtering through the above-mentioned filter membrane. The active substance-containing filtrate was processed by high performance liquid chromatography (HPLC) (column: YMC C18 of 50 mm diameter × 1000 mm length; mobile phase: water/methanol of 50/50 to 100/0; detection: UV 276 nm) to separate into two components, and antibiotics HUT57A and HUT57B were obtained.

(2)-3 Recovery and Purification:

The obtained culture was concentrated by filtering through a filter membrane (produced by Millipore Co.; 0.45 μm prostack) and washed fully with water, and 40 % isopropanol was added thereto to extract the active substance. The resulting extract was again filtered through a filter membrane (produced by Millipore Co.; fractionating molecular weight 3000 prostak). The active substance-containing filtrate was processed by high performance liquid chromatography (HPLC) (column: YMC C18 of 50 mm diameter × 1000 mm length; mobile phase: water/methanol of 50/50 to 100/0; detection: UV 276 nm) to separate into two components, and HUT57A and HUT57B were obtained.

The thus obtained HUT57A and HUT57B each are a white powder, having the above-mentioned physico-chemical properties. They are peptide antibiotics. Only two Iturin and Bacillomycin L are known up to the present, as antifungal peptide antibiotics to be produced by microorganisms of the genus Bacillus. HUT57A and B of the present invention are different from the known antibiotics in the physico-chemical properties such as the structure and the amino acid sequence to be derived therefrom and also in the effect. Therefore, they have been identified to be unknown novel substances.

Example 2

The antimicrobial activity of HUT57A and B as produced in Example 1 was measured to identify the antifungal property of them.

(1) The minimum growth inhibiting concentration (MIC) of HUT57A and B to fungi was measured on the basis of the method as designated by Japan Chemical Therapy Association, and the results shown in Table 4 below were obtained. As is understood from the results, the substances displayed broad and strong antifungal spectra to various fungi of from animal-infecting fungi to phytopathogenic fungi.

Table 4

| Antifungal Activity, in vitro, of HUT57A, B | | |
|---|---|---|
| Tested Fungi | MIC (μg/ml) | |
| | HUT57A | HUT57B |
| Cryptococcus neoformans IFO1320 | 6.0 | 5.2 |
| Candida albicans DUC1001 | | |
| yeast like form | 25.0 | 24.5 |
| filamentous form | 14.0 | 14.0 |
| Candida albicans DUC1002 | | |
| yeast like form | 25.0 | 23.5 |
| filamentous form | 14.0 | 14.0 |
| Candida tropicalis IFO3256 | 11.4 | 11.2 |
| Trichosporon cutaneum IFO1500 | 23.0 | 23.0 |
| Aspergillus fumigatus IAM2612 | 1.3 | 1.3 |
| Aspergillus flavas IAM2044 | 2.3 | 2.4 |
| Mucor guilliermondii IFO9403 | 1.2 | 1.2 |
| Trichophyton mentagrophytes IFO6124 | 10.0 | 10.0 |
| Trichophyton rubrum IFO9185 | 6.2 | 5.4 |
| Botrytis cinerea IFO31831 | 2.2 | 2.1 |
| Cochliobolus miyabeanus IFO5277 | 1.2 | 1.2 |
| Diaporothe citiri IFO9170 | 1.0 | 1.0 |
| Fusarium oxysporum HUT5015 | 1.2 | 1.2 |
| Fusarium solani ATCC11712 | 2.8 | 2.8 |
| Pyricularia oryzae IFO5279 | 1.3 | 1.3 |
| Gibberella zeae IFO7160 | 2.3 | 2.8 |
| Medium: Sabouraud-glucose-agar (pH 6.8) | | |

(2) Using a paper-disk method, the activity of HUT57A and B to bacteria was measured, and the results shown in Table 5 below were obtained. As is obvious from the results, HUT57A and B did not display any antibacterial activity to 14 strains of gram-positive and gram-negative bacteria even in a concentration of 100 µg/ml or more. Thus, it was verified that the antibiotics have an extremely high selective toxicity.

Table 5

| Bactericidal Activity of HUT57A, B by agar diffusion analysis | | |
|---|---|---|
| Tested Bacteria | Inhibited Zone (mm) (100 µg/disk) | |
| | HUT57A | HUT57B |
| Pseudomonas fluorescens JCM2479 | - | - |
| Pseudomonas cepacia JCM2800 | 1.0 | 1.0 |
| Escherichia coli JCM1465 | - | - |
| Bacillus brevis IFO12334 | - | - |
| Bacillus brevis JCM2505 | - | - |
| Bacillus cereus JCM2152 | - | - |
| Bacillus circulans JCM2504 | - | - |
| Bacillus coagulans JCM2257 | - | - |
| Bacillus licheniformis JCM2505 | - | - |
| Bacillus macerans JCM2500 | - | - |
| Bacillus megaterium JCM2506 | 1.4 | 1.0 |
| Bacillus polymyxa JCM2507 | 1.0 | 1.0 |
| Bacillus subtilis JCM1645 | 1.2 | 1.3 |
| Staphylococcus aureus JCM2413 | - | - |
| Medium: Müller-Hinton broth-agar (pH 7.0) | | |

The antifungal activity, in vitro, of HUT57A and B to Candida albicans DUC1001 was measured, by counting the number of the living cells. In both cases, the number of the living cells noticeably decreased just after the active substance was added thereto in a varying concentration of 25, 40 and 80 µg/ml. From the result, the substances each are considered to be a antifungal substance having an activity of killing the fungi. Since no natural antifungal substance having an activity of killing the fungi is unknown up to the present, the substances of the present invention were identified to be novel substances.

## Example 3

HUT57A and B as produced in Example 1 were tested with respect to the toxicity, by which the safety of them was verified.

(1) Cytotoxicity Test:

A floating suspension of CHU/IU cells, having a concentration of $1.3 \times 10^5$ cells/ml, was prepared, and this was separately put in a 96-well micro-plate in an amount of 100 µl/well. The cells were incubated at 37°C for 48 hours, and the medium was exchanged for 180 µl/well of a fresh medium. Then, HUT57 having a concentration of 10 mg/100 µl was diluted to 1/10 with the medium to have a maximum concentration. The diluted one having a maximum concentration was further stepwise diluted to 1/2, 1/4 . . . at regular intervals in four stages. 20 µl of the dilution having a varying concentration was added to each well and the cells therein were incubated for further 48 hours at 37°C. After 48 hours, MTT/PBS solution having a concentration of 5 mg/ml was diluted to 1/2 and 20 µl of the dilution was added to each well. After reacted for 4 hours, the plate was subjected to centrifugation at 1000 rpm for 5 minutes, 200 µl of the supernatant was removed, and 100 ml of 10 %Triton X-100 containing 0.04 N HCl/isopropanol was added to the residue. The amount of MTT formazan as eluted was measured with a micro-plate reader (by Corona Co.; MTP-120) (main wavelength 570 nm; side wavelength 630 nm).

Evaluation of the toxicity was effected in the manner mentioned below. Briefly, the average of the absorbance of the control well (Ac) and the average of the absorbance of the test well to which a varying concentration of HUT57 had been added (At) were obtained, and drug-sensitivity of [At/Ac] $\times$ 100 was obtained therefrom for evaluation of the toxicity. The value is a criterion of the viability of the tested cells. The results obtained are shown in Table 6 below. As is understood from the results, the substances of the present invention had no cytotoxicity to the incubated animal cells in a concentration of from 1 to 0.5 mg/ml or less and therefore the extremely high safety of them was verified.

Table 6 - Evaluation of Cytotoxicity

| Amount of Active Substance Added to Each Well (mg/ml) | 1.0 | 0.5 | 0.25 | 0.125 | 0.06125 | Control |
|---|---|---|---|---|---|---|
| Average Absorbance | 0.005 | 0.036 | 0.411 | 0.429 | 0.446 | 0.428 |
| [At/Ac] × 100 (%) | 1.2 | 8.4 | 96.0 | 100.2 | 104.2 | - |

(2) Acute Toxicity Test:

The freeze-dried products of HUT57A and B each from the culture broth obtained in Example 1-(1) were tested. Each product was dissolved in an injectable distilled water to prepare an aqueous 10 % (w/v)

solution of the product to be tested. The test solution was forcedly oral administered once to five male and five female ddy-N mice, after having been fasted for 13 hours, by the use of a stomach probe. The test ddy-N mice were about 4-week age ones and were obtained from Nippon Medico-Chemical Animal Sources Laboratories Co., Ltd. Prior to the test, all the test mice were preliminarily bred in the laboratory for one week so as to confirm the healthy condition of them. Then, the 5-week age mice were subjected to the test. The body weight of the test mice just before the test was 28 g (male mice) and from 18 to 22 g (female mice).

To each of the male and female mice, the test solution was administered in an amount of 20 ml per kg of the body weight, whereby 2,000 mg/kg of the test product was administered to them. Just after the administration, the vitality of all the test mice lowered slightly, but it came back to be normal in 2 hours after the administration. One hour after recovery of the vitality of them, they were fed, and the time-dependent mortality of the test mice was observed. The results obtained are shown in Table 7 below.

Table 7 - Time-Dependent Mortality of Test Mice

| Sex | Amount Administered (mg/kg) | Time-Dependent Mortality | | | Mortality (%) | $LD_{50}$ (mg/kg) |
|---|---|---|---|---|---|---|
| | | 5 hrs | 15 hrs | 1 2 3 4 5 6 ........14 days | | |
| Male Mice | 2,000 | 0/5 | 0/5 | 0/5..........................0/5 | 0 | >2,000 |
| Female Mice | 2,000 | 0/5 | 0/5 | 0/5..........................0/5 | 0 | >2,000 |

In the next test, mice of the same kind as mentioned above were used. Each of HUT57A and HUT57B was dissolved in DMSO (dimethylsulfoxide)-castor oil-5 % Glc solution, and the resulting solution was injected to the caudal vein of each of the five male mice once or plural times. For the single injection, the solution was administered to the mice each in an amount of 200 mg per kg of the body weight; and for the plural injections, it was administered to them 14 times each in an amount of 30 mg/kg/day. After the

injection(s), the time-dependent mortality of the test mice was observed and the results shown in Table 8 below were obtained.

Table 8 - Time-Dependent Mortality of Test Mice by Single Intravenous Injection

| Sex | Amount Administered (mg/kg) | Time-Dependent Mortality | | | Mortality (%) | $LD_{50}$ (mg/kg) |
|---|---|---|---|---|---|---|
| | | 5 hrs | 15 hrs | 1 2 3 4 5 6 .... 14 days | | |
| Male Mice | 200 | 0/5 | 0/5 | 0/5.....................0/5 | 0 | >200 |

Table 9 - Time-Dependent Mortality of Test Mice by Plural Intravenous Injections

| Sex | Amount Administered (mg/kg/day x times) | Time-Dependent Mortality | | | Mortality (%) |
|---|---|---|---|---|---|
| | | 5 hrs | 15 hrs | 1 2 3 4 5 6 ....... 14 days | |
| Male Mice | 30 x 14 | 0/5 | 0/5 | 0/5......................0/5 | 0 |

As is obvious from the above-mentioned results, oral administration of the test substances resulted in no death of the test mice, and the anatomical view of the test mice gave no abnormality. $LD_{50}$ of each of

the test substances as calculated by a probit method was 2000 mg/kg or more. The guide line by OECD (April 11, 1986) and others have defined that the maximum oral administration dose of a chemical substance to mice in the acute toxicity test is 2000 mg/kg, from which the low toxicity of the active substances of the present invention has been verified. $LD_{50}$ of each of HUT57A and HUT57B by single intravenous injection is 200 mg/ml or more, from which the low toxicity and safety of both the substances in injection have been verified like the case of oral administration of them.

Example 4

Tablets were formed from (1) 50 g of HUT57A, (2) 90 g of lactose, (3) 29 g of corn starch and (4) 1 g of magnesium stearate.

Precisely, (1) and (2) were mixed along with (3) (17 g), and the mixture was granulated along with a paste prepared from (3) (7 g). (3) (5 g) and (4) were added to the resulting granules and well blended, and the resulting blend was tabletted with a compression tabletting machine to produce 1000 tablets containing 50 mg/tablet of the active ingredient (1).

Example 5

65.0 parts of HUT57 (culture filtrate) as produced in Example 1, 30.0 parts of bentonite and 5.0 parts of sodium laurylsulfate were fully mixed to produce a wettable powder Of antifungal HUT57 for agricultural and horticultural use.

ADVANTAGE OF THE INVENTION

The present invention provides novel antibiotics HUT57A or/and B. Both HUT57A and B are unknown novel peptide antibiotics. The substances both have broad and strong antifungal spectra and are extremely useful for preventing and curing human and animal diseases caused by fungi, and they may be used in various administration routes of internal and external application, subcutaneous and intravenous application and others.

In addition, in accordance with the present invention, the substances may be formulated into agricultural antifungal with suitable agricultural carriers by ordinary methods, and they are extremely effectively be used as phytopathogenic antifungal. Moreover, since the present substances are derived from natural sources and therefore not only they gave no chemical injury to crop plants but also any particular injury to human, animals and fishes by them is not admitted. Therefore, they are especially excellent as low-toxic agricultural chemicals.

Reference to Deposited Microorganisms under Rule 13-2

1. Bacillus licheniformis UTK

   a. Name and Address of the Authorized Organ for Deposition of the Present Microorganisms:
      Name: Fermentation Research Institute of the Ministry of International Trade and Industry, Japan
      Address: No. 305, 1-3, Higashi 1-chome, Tsukuba, Ibaraki, Japan
   b. Date of Deposition in the Organ of a:
      May 9, 1990
   c. Deposition Number as Rendered by the Organ a:
      FERM BP-3794

SEQUENCE LISTING

(1) GENERAL INFORMATION:

(i) APPLICANT:
(A) NAME: Cabinet Fedit-Loriot (as patent attorneys)
(B) STREET: 38, avenue Hoche
(C) CITY: Paris
(E) COUNTRY: France
(F) POSTAL CODE (ZIP): 75008
(G) TELEPHONE: 1-42564235
(H) TELEFAX: 1-42898240
(I) TELEX: 642587 F

(A) NAME: Higeta Shoyu Co., Ltd.
(B) STREET: 2-3, Nihonbashikoamicho, Chuo-ku
(C) CITY: Tokyo
(E) COUNTRY: Japan
(F) POSTAL CODE (ZIP): 103

(ii) TITLE OF INVENTION: "NOVEL ANTIBIOTICS AND METHOD OF PRODUCING THEM AND USE OF THEM"

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(vi) PRIOR APPLICATION DATA:
(A) APPLICATION NUMBER: JP 4-103890
(B) FILING DATE: 31-MAR-1992

(vi) PRIOR APPLICATION DATA:
(A) APPLICATION NUMBER: JP 4-103954
(B) FILING DATE: 31-MAR-1992

(vi) PRIOR APPLICATION DATA:
(A) APPLICATION NUMBER: JP 4-103946
(B) FILING DATE: 31-MAR-1992

(2) INFORMATION FOR SEQ ID NO: 1:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(ix) FEATURE:
(A) NAME/KEY: Modified-site
(B) LOCATION: 1
(D) OTHER INFORMATION: /note= "Xaa represents a fatty alpha-amino acid"

(xi) SEQUENCE DESCRIPTION: SEQ ID NO: 1:

```
Xaa Asn Ser Pro Asn Tyr Asn Gln
1               5
```

```
(2) INFORMATION FOR SEQ ID NO: 2:

     (i) SEQUENCE CHARACTERISTICS:
          (A) LENGTH: 8 amino acids
          (B) TYPE: amino acid
          (C) STRANDEDNESS: single
          (D) TOPOLOGY: linear

    (ii) MOLECULE TYPE: peptide

    (ix) FEATURE:
          (A) NAME/KEY: Modified-site
          (B) LOCATION: 1
          (D) OTHER INFORMATION: /note= "Xaa represents
               2-aminotetradecanoic acid"

    (xi) SEQUENCE DESCRIPTION: SEQ ID NO: 2:

     Xaa Asn Ser Pro Asn Tyr Asn Gln
     1               5

(2) INFORMATION FOR SEQ ID NO: 3:

     (i) SEQUENCE CHARACTERISTICS:
          (A) LENGTH: 8 amino acids
          (B) TYPE: amino acid
          (C) STRANDEDNESS: single
          (D) TOPOLOGY: linear

    (ii) MOLECULE TYPE: peptide

    (ix) FEATURE:
          (A) NAME/KEY: Modified-site
          (B) LOCATION: 1
          (D) OTHER INFORMATION: /note= "Xaa represents
               2-aminopentadecanoic acid"

    (xi) SEQUENCE DESCRIPTION: SEQ ID NO: 3:

     Xaa Asn Ser Pro Asn Tyr Asn Gln
     1               5
```

## Claims

**1.** Novel compounds of a formula:
X-Asn-Ser-Pro-Asn-Tyr-Asn-Gln
where X represents an $\alpha$-amino-fatty acid (see SEQ ID No: 1).

**2.** Antifungal antibiotics of a formula:
X-Asn-Ser-Pro-Asn-Tyr-Asn-Gln
where X represents an $\alpha$-amino-fatty acid (see SEQ ID No: 1).

**3.** A novel antibiotic HUT57A having the following physico-chemical properties:
1)

| | |
|---|---|
| Elementary Analysis: | C: 55.1<br>H: 7.2<br>N: 16.1 |

2) Melting Point: 128 ° C
3) Specific Rotatory Power: $[\alpha]^{20}_{D}$ -79.3 (methanol)
4) Ultraviolet Absorption Spectrum: Fig. 1
5) Infrared Absorption Spectrum: Fig. 2
6) This is a compound to be represented by a formula:
X-Asn-Ser-Pro-Asn-Tyr-Asn-Gln
where X represents an $\alpha$-amino-fatty acid of a formula:

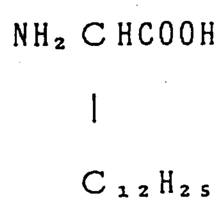

(see SEQ ID No: 2).

**4.** A novel antibiotic HUT57B having the following physico-chemical properties:
1)

| | |
|---|---|
| Elementary Analysis: | C: 55.6<br>H: 7.3<br>N: 15.7 |

2) Melting Point: 128 ° C
3) Specific Rotatory Power: $[\alpha]^{20}_{D}$ -78.5 (methanol)
4) Ultraviolet Absorption Spectrum: Fig. 3
5) Infrared Absorption Spectrum: Fig. 4
6) This is a compound to be represented by a formula:
X-Asn-Ser-Pro-Asn-Tyr-Asn-Gln
where X represents an $\alpha$-amino-fatty acid of a formula:

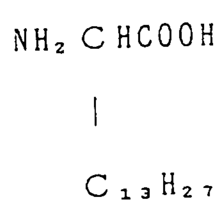

(see SEQ ID No: 3).

**5.** A method of producing novel antibiotic(s) HUT57A or/and B, in which microorganisms of belonging to the genus Bacillus and producing novel antibiotic(s) HUT57A or/and B are incubated and novel antibiotic(s) HUT57A or/and B is/are collected from the culture.

**6.** The method as claimed in claim 5, in which the microorganisms of belonging to the genus Bacillus and producing novel antibiotic(s) HUT57A or/and B are Bacillus licheniformis UTK.

**7.** An antifungal containing novel antibiotic(s) HUT57A or/and B as an active ingredient.

**8.** An antifungal containing a culture of microorganisms of belonging to the genus Bacillus and producing novel antibiotic(s) HUT57A or/and B or the processed product of the culture, as an active ingredient.

**9.** The antifungal as claimed in claim 8, in which the processed product of the culture is condensate, paste and/or dry powder of the cells and/or the culture.

**10.** The antifungal as claimed in claim 7, 8 or 9, which is applied to human and animals and to agricultural and horticultural use.

FIG. 1

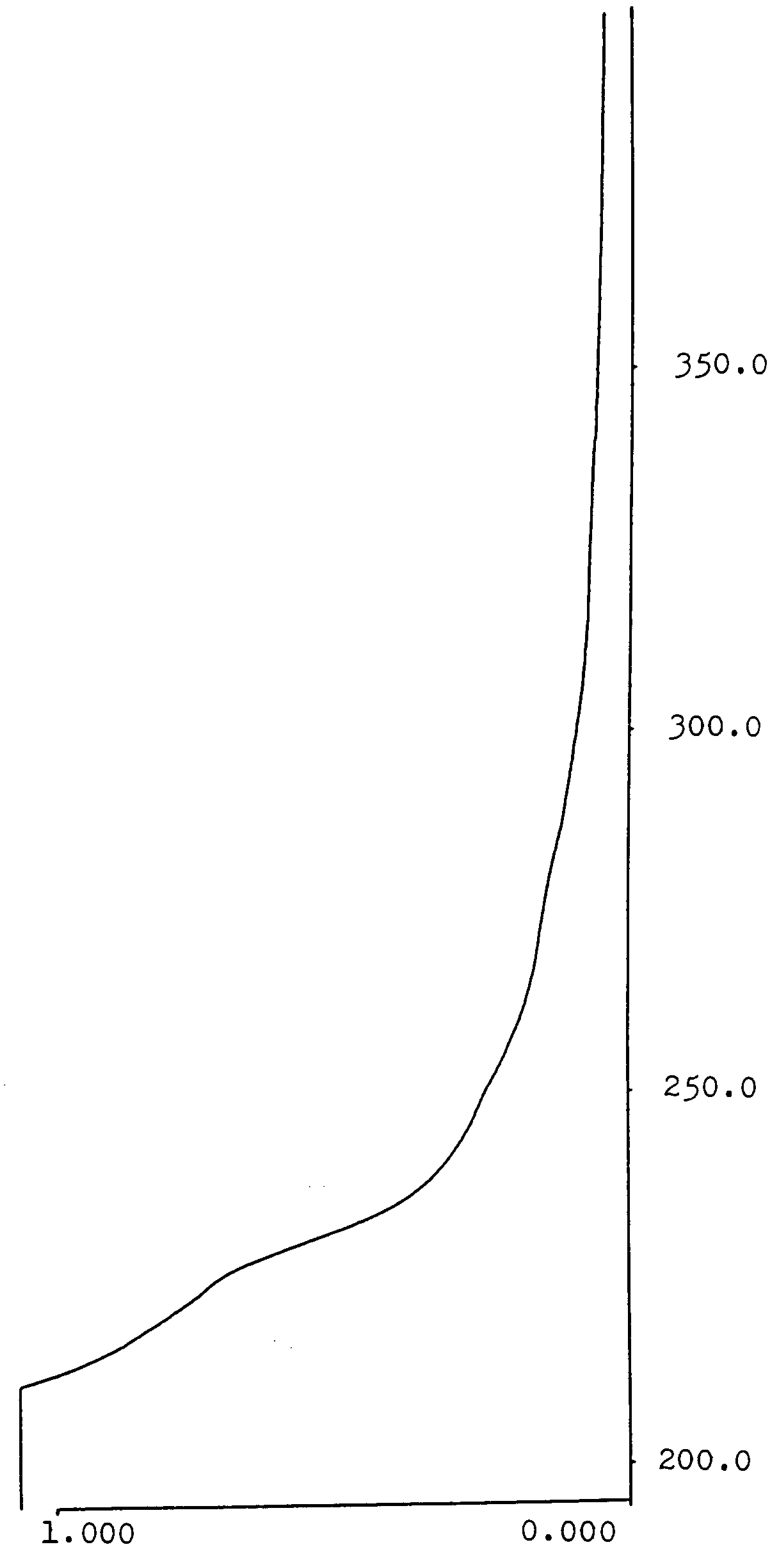

FIG. 2

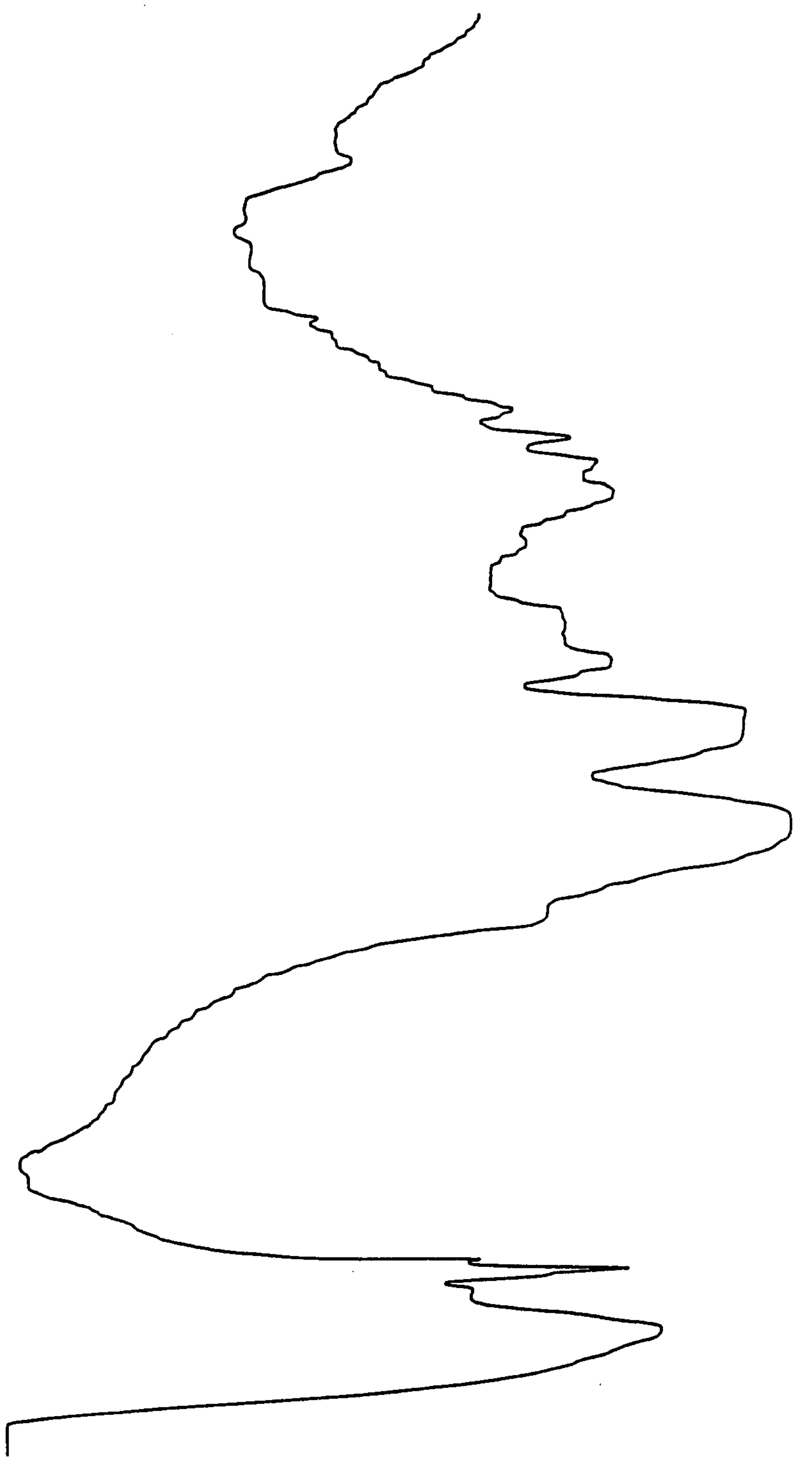

FIG. 3

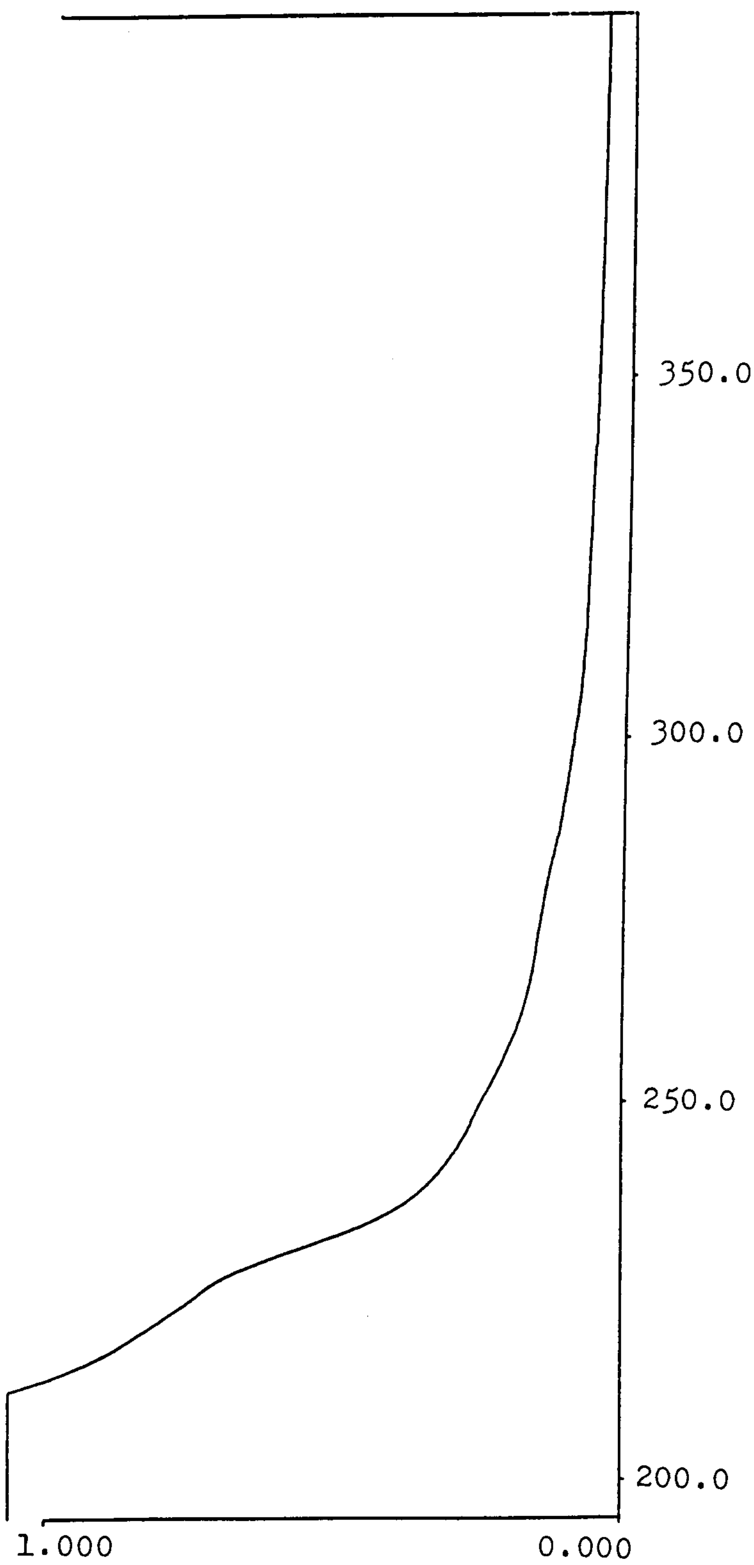

FIG. 4

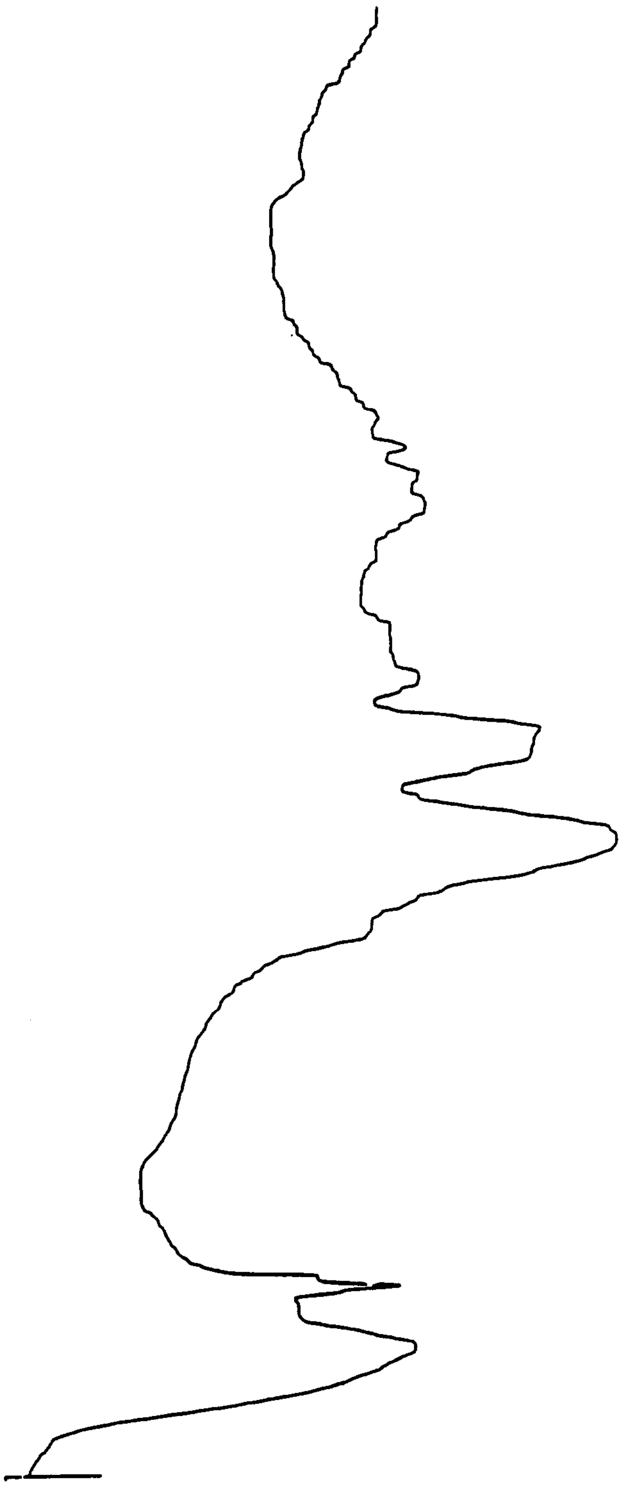

# INTERNATIONAL SEARCH REPORT

International application No.
PCT/JP93/00246

**A. CLASSIFICATION OF SUBJECT MATTER**

Int. $Cl^5$ C07K15/04, A61K37/02, C12P21/02//(C12P21/02, C12R1:10)

According to International Patent Classification (IPC) or to both national classification and IPC

**B. FIELDS SEARCHED**

Minimum documentation searched (classification system followed by classification symbols)

Int. $Cl^5$ C07K15/00, A61K37/02, C12P21/00

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| A | JP, A, 2-22299 (Agricultural Genetics Co.,Ltd.) January 25, 1990 (25. 01. 90), & US, A, 5061495 & EP, A2, 332369 | 1-10 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| May 20, 1993 (20. 05. 93) | June 8, 1993 (08. 06. 93) |
| Name and mailing address of the ISA/<br>Japanese Patent Office<br>Facsimile No. | Authorized officer<br><br>Telephone No. |

Form PCT/ISA/210 (second sheet) (July 1992)